# EUROPEAN PATENT APPLICATION

(11) **EP 3 354 194 A2**
(43) Date of publication of application: **01.08.2018**
(21) Application number: 17186238.6
(22) Date of filing: 15.08.2017
(51) Int. Cl.: A61B 5/021

(54) **BLOOD PRESSURE MEASUREMENT DEVICE AND METHOD**

(30) Priority: 25.01.2017 TW 106103043
(71) Applicant: Maisense Inc., Zhubei City 30273 (TW)
(72) Inventor: Lee, Yung-Pin, 30273 Zhubei City (TW); Lee, Min-Hao, 30273 Zhubei City (TW)
(74) Representative: dompatent von Kreisler Selting Werner - Partnerschaft von Patent- und Rechtsanwälten mbB

(57) **Abstract**

A blood pressure measurement device includes a body, a film-type pressure sensing unit, a first electrode, and a second electrode. The body includes a pulse sensing surface and a concave portion. The film-type pressure sensing unit is disposed on the pulse sensing surface and communicated with the concave portion. The first electrode is disposed on the pulse sensing surface. The second electrode is disposed on the body corresponding to the first electrode. The first electrode and second electrode are a pair of ECG electrodes. A blood pressure measurement method cooperated with the blood pressure measurement device is also disclosed.

## Description

### BACKGROUND OF THE INVENTION

### Field of Invention

The present disclosure relates to a blood pressure measurement device and a blood pressure measurement method.

### Related Art

The blood pressure is one of important indicators for the observation of the individual's health condition. The abnormal value of the blood pressure, either the too-high or too-low value, implies that a problem of the individual's physiological condition, especially the cardiovascular disease that is difficult to be observed from the outlook of the individual, arises. Because the elder's body has the aging function, there is a high risk of suffering from the cardiovascular disease, and it is a very important work to regularly measure the blood pressure of the elder so that the disease can be detected and treated early. However, the modem human beings have the high life pressure, and the irregular three meals. Thus, the group of persons whose blood pressures need to be regularly monitored has the decreasing age.

The conventional sphygmomanometer generally includes a cuff, a host and a rubber tube. Upon measurement of the blood pressure, the cuff is wear on the arm of the user, and the host controls the inflating pressurization and the deflating depressurization on the cuff through the rubber tube and substitutes the pressure fluctuation value, which represents vibration amplitude of the blood vessel wall and is sensed by the pressure sensor contained in the cuff, into the oscillometry to calculate the blood pressure.

However, the conventional sphygmomanometer tends to make the user feel uncomfortable in the pressurization and depressurization processes, needs a too-long measurement time, and may hurt the older user's weak skin, so that the user's measurement desire is low. Besides, it is disadvantageous to the long-term use. In addition, the complicated operation procedure is very difficult to the new user, especially the elder people. Moreover, regarding the portable property, the sizes of the host and the cuff are too large and the weights thereof are too heavy, so it is disadvantageous to the monitoring of the blood pressure at any time.

Recently, some watch-type blood pressure measurement devices are available in the market. Although this new product has overcome the problem of the portable property of the conventional sphygmomanometer 1, the minimized product cannot be properly fixed at the measurement location and thus cannot precisely measure the pulse signal of the blood vessel. Thus, the blood pressure measurement value is not precise, or even the measurement failure may occur.

Therefore, it is a great need to provide a blood pressure measurement device that can be easily carried, has the rapid measurement process without causing the uncomfortable problem upon pressurization and depressurization, and can be easily and precisely fixed at the measurement location to enhance the accuracy and success rate as measuring the blood pressure.

### SUMMARY OF THE INVENTION

In view of the foregoing, an objective of the disclosure is to provide a blood pressure measurement device and a blood pressure measurement method that do not need the cuff for pressurization and depressurization. Accordingly, the size of the device can be minimized to carry out the easy carrying issue and enhance the convenience in measuring the blood pressure so as to achieve the purpose of monitoring the blood pressure at any time. More important, the concave portion is communicated with the pulse sensing surface, so that the user can easily and precisely find the pulse position (assisted by finger) for measurement so as to place the film-type pressure sensing unit on the measuring position. In some embodiments, the present disclosure can easily place the device on the measuring position when measuring the signals. This is an intuitional operation for precisely measuring the blood pressure, and the operation procedure is simple so that the users of any ages can easily operate it.

To achieve the above objective, the present disclosure discloses a blood pressure measurement device, which includes a body, a film-type pressure sensing unit, a first electrode and a second electrode. The body includes a pulse sensing surface and a concave portion. The film-type pressure sensing unit is disposed on the pulse sensing surface and communicated with the concave portion. The first electrode is disposed on the pulse sensing surface, and the second electrode is disposed on the body corresponding to the first electrode. The first electrode and second electrode are a pair of ECG electrodes.

In addition, the present disclosure also discloses a blood pressure measurement method cooperated with a blood pressure measurement device. Herein, the blood pressure measurement device includes a body, a film-type pressure sensing unit, a first electrode and a second electrode. The body includes a pulse sensing surface and a concave portion. The film-type pressure sensing unit is disposed on the pulse sensing surface and communicated with the concave portion. The first electrode is disposed on the pulse sensing surface, and the second electrode is disposed on the body corresponding to the first electrode. The first electrode and second electrode are a pair of ECG electrodes. The blood pressure measurement method includes the following steps of: contacting the film-type pressure sensing unit with a human body; inserting at least one finger into the concave portion to press the film-type pressure sensing unit for sensing a pulse of the human body; continuously moving the blood pressure measurement device and contacting the film-type pressure sensing unit with the human body until the film-type pressure sensing unit retrieves at least one pulse signal; obtaining an electrocardio signal by the first electrode and the second electrode; and obtaining a blood pressure value according to the electrocardio signal and the pulse signal.

In one embodiment, the blood pressure measurement device further includes a calibration electrode disposed on the body.

In one embodiment, the calibration electrode and the second electrode are disposed at two sides of the pulse sensing surface, respectively.

In one embodiment, the second electrode is disposed on an inner wall of the concave portion.

In one embodiment, the second electrode is disposed adjacent to at least one side of the pulse sensing surface.

In one embodiment, an inner wall of the concave portion includes a slant surface.

In one embodiment, a waterproof material is configured on an inner wall of the concave portion.

In one embodiment, the first electrode and the film-type pressure sensing unit are overlapped, and an area of the first electrode is greater than an area of the film-type pressure sensing unit.

In one embodiment, the first electrode is disposed around the film-type pressure sensing unit.

In one embodiment, the body further includes a display unit disposed on the pulse sensing surface.

In one embodiment, the film-type pressure sensing unit includes a strain gauge or a piezoelectric element.

In one embodiment, when the film-type pressure sensing unit contacts with the human body, the first electrode also contacts with the human body.

In one embodiment, when a user holds the blood pressure measurement device by a hand, the second electrode is in contact with the hand of the user.

In one embodiment, when the first electrode and the second electrode obtain the electrocardio signal, the blood pressure measurement device is immediately enabled to measure the pulse signal.

In one embodiment, after the film-type pressure sensing unit senses the pulse signal, the blood pressure measurement device is immediately enabled to measure the electrocardio signal.

In one embodiment, the body is configured with a power switch, which is turned on for measuring the pulse signal and the electrocardio signal.

As mentioned above, the blood pressure measurement device and method of the disclosure can measure the pulse signal and the electrocardio signal for calculating the blood pressure value without utilizing the cuff for pressurization and depressurization. Accordingly, the size of the device can be minimized to carry out the easy carrying issue and enhance the convenience in measuring the blood pressure so as to achieve the purpose of monitoring the blood pressure at any time. More important, the concave portion is communicated with the pulse sensing surface, so that the user can easily and precisely find the pulse position (assisted by finger) for measurement so as to place the film-type pressure sensing unit on the measuring position. In some embodiments, the present disclosure can easily place the device on the measuring position when measuring the signals. This is an intuitional operation for precisely measuring the blood pressure, and the operation procedure is simple so that the users of any ages can easily operate it.

### BRIEF DESCRIPTION OF THE DRAWINGS

The invention will become more fully understood from the detailed description and accompanying drawings, which are given for illustration only, and thus are not limitative of the present invention, and wherein:
FIG. 1 is a schematic diagram showing a blood pressure measurement device according to an embodiment of the disclosure;
FIG. 2 is a block diagram of the blood pressure measurement device according to the embodiment of the disclosure;
FIG. 3A is a schematic diagram showing a finger contacting the film-type pressure sensing unit according to the embodiment of the disclosure;
FIG. 3B is a schematic enlarged diagram of the oval area S of FIG. 3A containing the first electrode and the film-type pressure sensing unit according to the embodiment of the disclosure;
FIG. 3C is another schematic enlarged diagram of the oval area S of FIG. 3A containing the first electrode and the film-type pressure sensing unit according to the embodiment of the disclosure;
FIG. 4 is a schematic diagram showing a finger contacting the film-type pressure sensing unit according to the embodiment of the disclosure;
FIG. 5A is a schematic diagram showing the second electrode and the calibration electrode according to the embodiment of the disclosure;
FIG. 5B is another schematic diagram showing the second electrode and the calibration electrode according to the embodiment of the disclosure;
FIG. 5C is another schematic diagram showing the second electrode and the calibration electrode according to the embodiment of the disclosure;
FIG. 6 is a schematic diagram (viewing from another side) showing the blood pressure measurement device according to an embodiment of the disclosure;
FIG. 7 is a flow chart of a blood pressure measurement method according to an embodiment of the disclosure; and
FIG. 8 is a schematic diagram showing that the user moves the blood pressure measurement device to find the pulse.

### DETAILED DESCRIPTION OF THE INVENTION

The present invention will be apparent from the following detailed description, which proceeds with reference to the accompanying drawings, wherein the same references relate to the same elements.

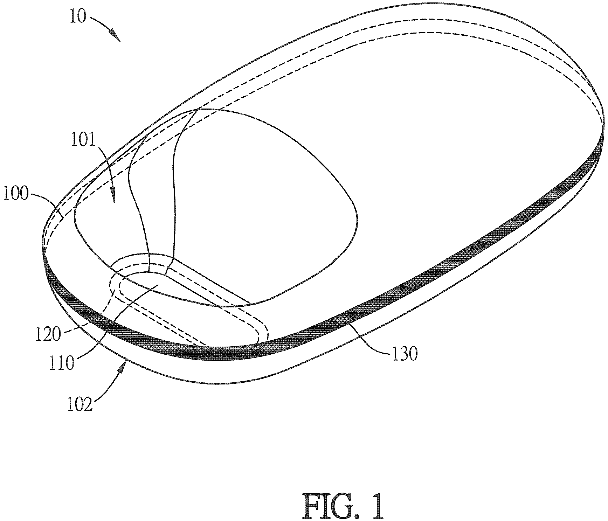

FIG. 1 is a schematic diagram showing a blood pressure measurement device 10 according to an embodiment of the disclosure. As shown in FIG. 1, the blood pressure measurement device 10 includes a body 100, a film-type pressure sensing unit 110, a first electrode 120, and a second electrode 130.

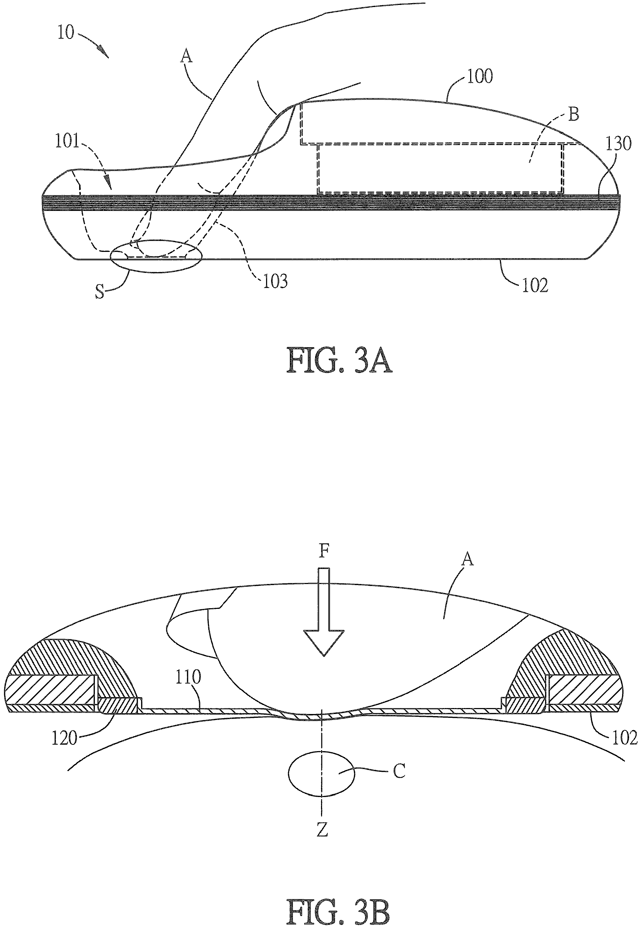

Referring to FIGS. 1 and 3A, the body 100 includes a concave portion 101 and a pulse sensing surface 102. In this embodiment, the body 100 further includes a housing for accommodating the electronic elements of the blood pressure measurement device 10. The housing can be designed as any shape. In this embodiment, the housing has an ergonomic curved upper surface, a lower planar surface, and an annular side surface. The blood pressure measurement device 10 can be a hand-held device. In the view of the user when operating the blood pressure measurement device 10, the concave portion 101 is a recess from the top surface of the body 100, so that the user's hand can put on the top surface of the body 100 to hold the blood pressure measurement device 10 and insert the finger A (e.g. the index finger, middle finger or ring finger) into the concave portion 101 simultaneously.

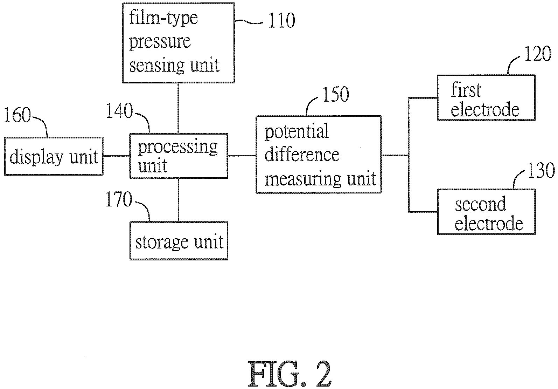

FIG. 2 is a block diagram of the blood pressure measurement device 10 according to the embodiment of the disclosure. The body 100 further accommodates the units or elements of the blood pressure measurement device 10. In this embodiment, the blood pressure measurement device 10 further includes a processing unit 140, a potential difference measuring unit 150, a display unit 160 and a storage unit 170. The processing unit 140 is electrically connected to the film-type pressure sensing unit 110, the potential difference measuring unit 150, the display unit 160 and the storage unit 170. The potential difference measuring unit 150 is electrically connected to the first electrode 120 and the second electrode 130. The processing unit 140 accesses the storage unit 170 to retrieve the required program instructions and data, and performs data computation according to the program instructions to obtain a blood pressure value and to control other units to operate.

FIG. 3A is a schematic diagram showing a finger contacting the film-type pressure sensing unit 110 according to the embodiment of the disclosure. As shown in FIG. 3A, the inner wall of the concave portion 101 includes a slant surface 103. In more detailed, the concave portion 101 is a recess from the top surface, and when the user inserts his/her finger A into the concave portion 101, the slant surface 103 can properly support the finger A. In this case, any of the inner walls of the concave portion 101 can be a slant surface 103, and the slant surface 103 can extend to the bottom of the concave portion 101. In one embodiment, the inner wall (including the bottom) of the concave portion 101 is configured with a waterproof material for blocking the dusts and water in operation, thereby extending the lifetime of the blood pressure measurement device 10. The waterproof material can be a waterproof nano-coating or silica gel. In this embodiment, a waterproof silica gel is applied on the top surface of the body 100 and extends to the inner wall of the concave portion 101.

The pulse sensing surface 102 is configured to contact and measure the pulse and is located under the body 100. The film-type pressure sensing unit 110 is disposed on the pulse sensing surface 102 and communicated or connected with the concave portion 101. In practice, the film-type pressure sensing unit 110 is located at the junction of the concave portion 101 and the pulse sensing surface 102. The finger A can be inserted into the concave portion 101 and directly or indirectly contacted (separated by the waterproof material) with the film-type pressure sensing unit 110.

The film-type pressure sensing unit 110 is disposed on the pulse sensing surface 102 and communicated with the concave portion 101. Besides, the film-type pressure sensing unit 110 may include at least one or more strain gauge, a piezoelectric element or any other component that can sense the pulse. Preferably, the film-type pressure sensing unit 110 further includes an insulation coating for covering the element, and the thickness of the insulation coating must be very thin (e.g. less than 1.5 mm). In this embodiment, the thickness of the entire film-type pressure sensing unit 110 is between 0.6 mm and 1.1 mm, and is preferably 1.0 mm. To be noted, this configuration must still allow the finger A to insert into the concave portion 101, and the user is capable of sensing the vibration of the pulse sensing surface 102. Accordingly, the user can use his/her finger (or other portions of the human body) to find the pulse through the film-type pressure sensing unit 110. In this embodiment, the film-type pressure sensing unit 110 is a strain gauge, which is composed of a piezoresistive material (e.g. a metal sheet) and an insulation substrate. In other embodiments, the film-type pressure sensing unit 110 can be made of piezoelectric material, which is light and thin and allows the user to sense the vibration of the pulse sensing surface 102 in the concave portion 101 so as to sense the pulse signal. As mentioned in the above embodiment, the inner wall of the concave portion 101 is configured with the waterproof material, such as silica gel or resin film, for covering the elements in the film-type pressure sensing unit 110. The total thickness from the waterproof material to the pulse sensing surface 102 is 1.0-2.4 mm. To be noted, this configuration must still allow the user to sense the vibration of the pulse sensing surface 102 through the concave portion 101 and sense the pulse signal.

As shown in FIG. 3A, the blood pressure measurement device 10 is powered by a battery B. In this embodiment, the battery B is an AA battery. To be noted, the battery B can be any shape or size of battery, such as an AAA battery, a button battery or the likes, and it can be an alkaline battery or carbon zinc battery. Alternatively, the body 100 can be embedded with a lithium battery and an USB port, and the lithium battery can be recharged through the USB port. This disclosure is not limited.

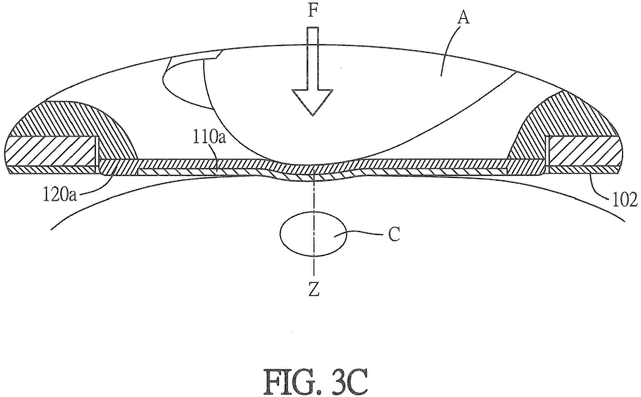

FIGS. 3B and 3C are schematic enlarged diagrams of the oval area S of FIG. 3A in different aspects containing the first electrode and the film-type pressure sensing unit according to the embodiment of the disclosure. The finger A of the user is placed on the film-type pressure sensing unit 110 and it applies a force F to push the blood pressure measurement device 10 against a human body (skin) of the user. The finger A and the vessel C to be measured are at least partially overlapped in the projection direction Z. As shown in FIG. 3B, the first electrode 120 is an electrocardiography (ECG) electrode, which is cooperated with the second electrode 130 to measure the electrocardio signal of the user. The first electrode 120 is made of an electric conductive material and disposed around the film-type pressure sensing unit 110 (e.g. a ring). The first electrode 120 and the film-type pressure sensing unit 110 are disposed on the pulse sensing surface 102. Accordingly, when the pulse sensing surface 102 contacts against the human body to be measure, the first electrode 120 and the film-type pressure sensing unit 110 can both contact with the human body to be measure. In this embodiment, the shape of the first electrode 120 and the film-type pressure sensing unit 110 can be, for example but not limited to, oval, circular, square, or irregular. In another embodiment, as shown in FIG. 3C, the first electrode 120a is a film electrode, and is overlapped with the film-type pressure sensing unit 110a. The area of the first electrode 120a can be greater than the area of the film-type pressure sensing unit 110a. The film-type pressure sensing unit 110a is under the first electrode 120a and is close to the human body to be measured. The first electrode 120a is above the film-type pressure sensing unit 110a, and an insulation layer (not shown) is configured between the first electrode 120a and the film-type pressure sensing unit 110a for preventing the interference of the first electrode 120a and the film-type pressure sensing unit 110a. In this embodiment, the film-type pressure sensing unit 110a includes an insulation substrate, so the additional insulation layer is not needed. Since the first electrode 120a is a film electrode (preferably thinner than 0.2 mm), the operation of finding the pulse by the user through the film-type pressure sensing unit 110a and the first electrode 120a is not interfered by the first electrode 120a.

Referring to FIGS. 1 and 2, the second electrode 130 is disposed on the body 100 and is located corresponding to the first electrode 120. The second electrode 130 can be disposed on the side surface of the body 100, the inner wall of the concave portion 101, or any place of the body 100 that can be easily touched by the user. In this embodiment, the second electrode 130 is disposed on the annular side surface of the body 100, so that it is shaped as an annular electrode. The first electrode 120 and the second electrode 130 are a pair of ECG electrodes. One of the first electrode 120 and the second electrode 130 is a positive electrode, and the other one is a negative electrode. The first electrode 120 and the second electrode 130 are electrically connected to the potential difference measuring unit 150 disposed in the body 100. By contacting with different parts of the human body, the first electrode 120 and the second electrode 130 can form a set of leads. When the heart is performing the depolarization activity, the potential difference measuring unit 150 can measure a potential difference between the electrodes, and the processing unit 140 converts the potential difference to obtain an electrocardio signal. In this embodiment, the first electrode 120 contacts the left-hand's wrist of the user, and the second electrode 130 contacts the finger (thumb) of user's right hand to form a loop through the heart. Specifically speaking, the limb lead electrocardio signal is a first limb lead (lead I) serving as a single lead electrocardio signal. In this embodiment, the electrocardio signal is an electrocardiography (ECG) signal. However, it is to be noted that, in other embodiments of the invention, the first electrode 120 and the second electrode 130 may also contact the other parts of the human body, such as the left and right legs, the part near the rib of the trunk or the part near the armpit, to measure the electrocardio signals of other leads. However, the disclosure is not restricted thereto.

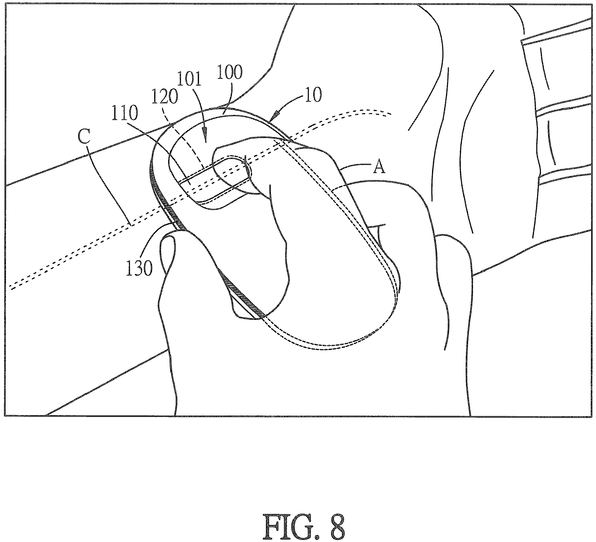

As shown in FIG. 3A, when operating the blood pressure measurement device 10 of the disclosure, the index finger of the right hand is inserted into the concave portion 101, and the thumb and middle fingers clip the side surface of the body 100 (e.g. the annular side surface as shown in FIG. 8). Thus, the user can hold and move the blood pressure measurement device 10 by one hand, and a part of palm can rest on the top surface of the body 100 (e.g. the curved upper surface of the case). As shown in FIG. 3A, the oval area S shows the junction of the bottom of the concave portion 101 and the film-type pressure sensing unit 110. The finger A is inserted into the concave portion 101 and contacted with the film-type pressure sensing unit 110, and the film-type pressure sensing unit 110 is pressed to contact against a part of the human body to be measured so as to obtain the pulse. In this case, the sensitive fingertip of the finger A presses the film-type pressure sensing unit 110, and the film-type pressure sensing unit 110 is held by one hand and moved to the part of the human body to be measured.

Since the pulse sensing surface 102 is disposed at the bottom of the body 100, the user is hard to precisely align the film-type pressure sensing unit 110, which is disposed on the pulse sensing surface 102, to the part of the human body to be measured, such as the vessel C at the inside portion of the wrist or a position with thinner skin. In this embodiment, the finger A can insert into the concave operation 101 and contact with the film-type pressure sensing unit 110, so that the user can feel the vibration of the pulse through the film-type pressure sensing unit 110. In other words, the user can insert the finger A into the concave portion 101 to find a part of the human body for easy pulse measurement. If the use cannot sense the pulse, he/she can move the blood pressure measurement device 10 to find another part of the human body with an obvious pulse or vibration. This operation can speed the entire measurement procedure and increase the accuracy of the blood pressure measurement.

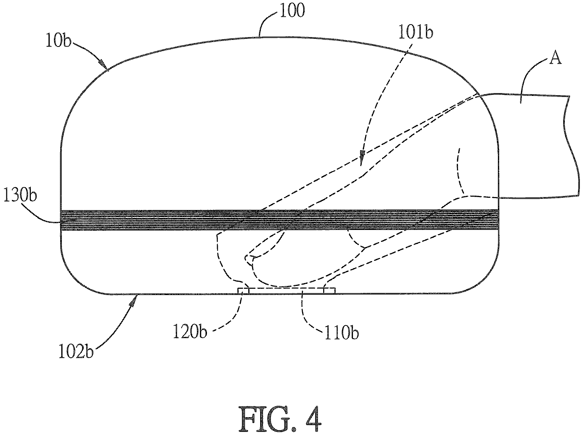

In the blood pressure measurement device 10b of another embodiment as shown in FIG. 4, the pulse sensing surface 102b is disposed at the bottom of the body 100b, and the concave portion 101b is a recess from a side surface of the body 100b and communicated with the film-type pressure sensing unit 110b. The user can insert the index finger, middle finger or ring finger into the concave portion 101b to contact or press the film-type pressure sensing unit 110b. In this case, the user inserts the index finger into the concave portion 101b to contact or press the film-type pressure sensing unit 110b, and holds two sides of the body 100b by the thumb and middle finger. Only the position of the concave portion 101b is different from the configuration of the embodiment shown in FIG. 1. To be noted, the film-type pressure sensing unit 110b must be disposed on the pulse sensing surface 102b, and communicated with the concave portion 101b.

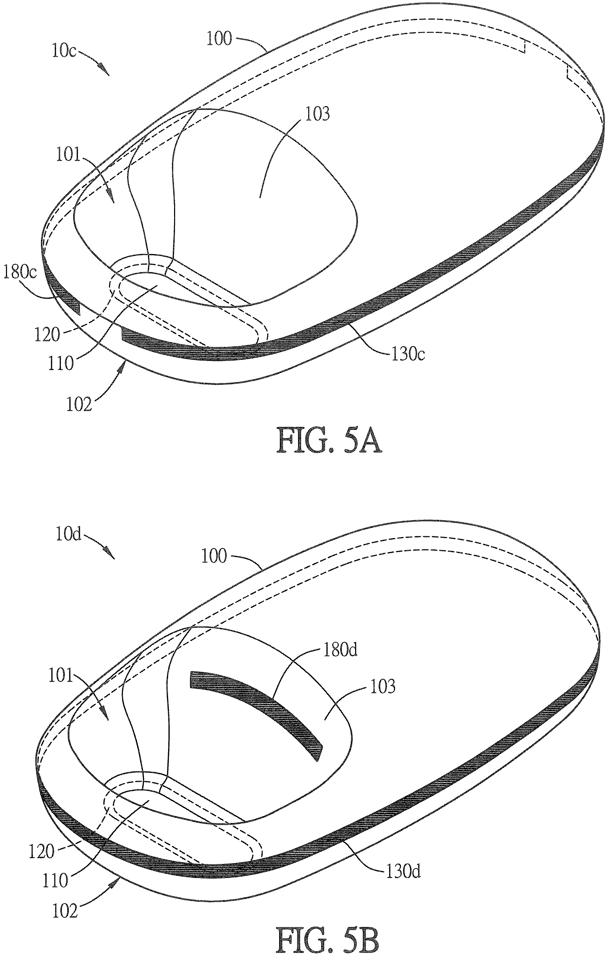

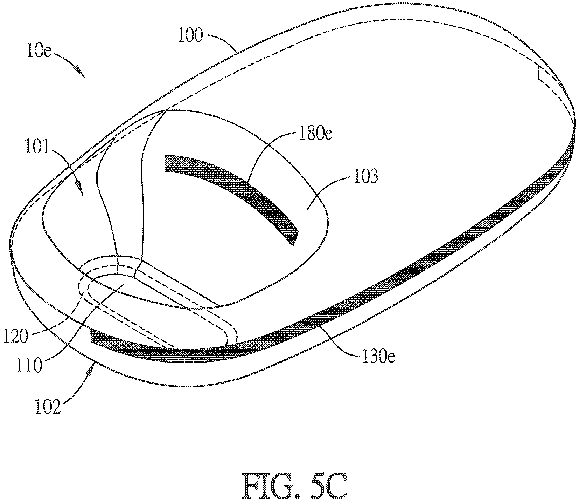

FIGS. 5A to 5C are schematic diagrams showing the second electrode and the calibration electrode of different aspects according to the embodiment of the disclosure. Different from the above-mentioned blood pressure measurement device 10, the blood pressure measurement device 10c of this embodiment further includes a calibration electrode 180c for filtering undesired interference noise, or generating a calibration value with the first electrode 120 to produce an electrocardio signal average with the first electrode 120 and the second electrode 130c, thereby obtaining a more accurate electrocardio signal. In the embodiment, the calibration electrode 180c is electrically connected with the potential difference measuring unit 150. As shown in FIG. 5A, the first electrode 120 is disposed on the pulse sensing surface 102, and the second electrode 130c and the calibration electrode 180c are disposed on two sides of the annular side surface of the body 100, respectively. The second electrode 130c and the calibration electrode 180c are curved electrodes and disposed opposite to each other. When the user holds the blood pressure measurement device 10c, the thumb and the middle finger (or ring finger) are placed on the second electrode 130c and the calibration electrode 180c, respectively. Different from the blood pressure measurement device 10, in another embodiment as shown in FIG. 5B, the second electrode 130d is disposed on the annular side surface of the body 100, and the calibration electrode 180d is disposed on the inner wall of the concave portion 101. The positions of these two components can be switched, and the measurement result is not affected. As shown in FIG. 5B, the second electrode 130d can cover the entire side surface of the body 100. Otherwise, as shown in FIG. 5C, the second electrode 130e is only cover a part (one side) of the side surface of the body 100. In practice, the first electrode, the second electrode, and the calibration electrode can be disposed on any position of the body that allows the part of the human body to be measured (usually the hand) to contact all of these electrodes. The electrode can be disposed on any position of the inner wall of the concave portion 101 that can be contacted by the inserted finger A. In this embodiment, as shown in FIG. 5C, the inner wall of the concave portion 101 further includes a slant surface 103, and the calibration electrode 180e is disposed laterally on the slant surface 103 with a height (from the bottom of the concave portion 101) of about one or two knuckles. In the above configuration, the fingers can easily contact with the film-type pressure sensing unit 110 and the electrodes. Referring to the situation of FIG. 3A, the slant surface 103 is configured for resting the inserted finger A. The measurement procedure can be easier with the supporting of the slant surface 103. Besides, the user can stay in the convertible position longer, so that the first electrode 120, the second electrode 130e and the calibration electrode 180e can perform more precise measurement or calibration operation.

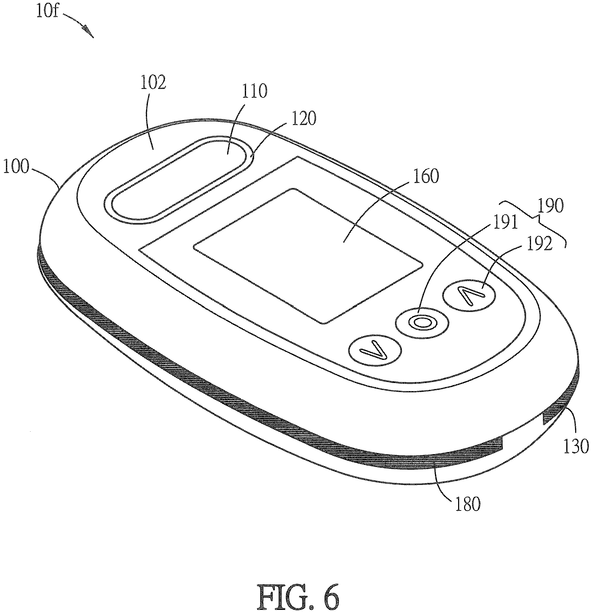

FIG. 6 is a schematic diagram (viewing from another side) showing the blood pressure measurement device 10f according to an embodiment of the disclosure. In one embodiment, the blood pressure measurement device 10f further includes a display unit 160, and the display unit 160 and the input unit 190 are disposed on the pulse sensing surface 102. The display unit 160 receives the measuring result of the processing unit 140 (e.g. the blood pressure and the pulse) and displays the result. Accordingly, after the user finishes the blood pressure measurement, he/she can simply turn over the blood pressure measurement device 10f to immediately view the measuring results such as the blood pressure value. This design is intuitive and very convenient. Of course, in other embodiments, the blood pressure measurement device 10f may further include a communication unit for transmitting the measuring result to the smart phone or tablet computer for displaying through wireless communication technology such as Bluetooth, 3G/4G mobile communication protocol, or Wi-Fi. This disclosure is not limited. In addition, the body 100 of the blood pressure measurement device 10f may be further configured with an input unit 190. The input unit 190 at least includes a power switch 191 and a pair of adjusting buttons 192. The power switch 191 is configured for enabling or disabling the entire system of the blood pressure measurement device 10f. The pair of adjusting buttons 192 may include a "+" button and a "-" button for the user to input the personal physiological information such as height, weight, age and the likes.

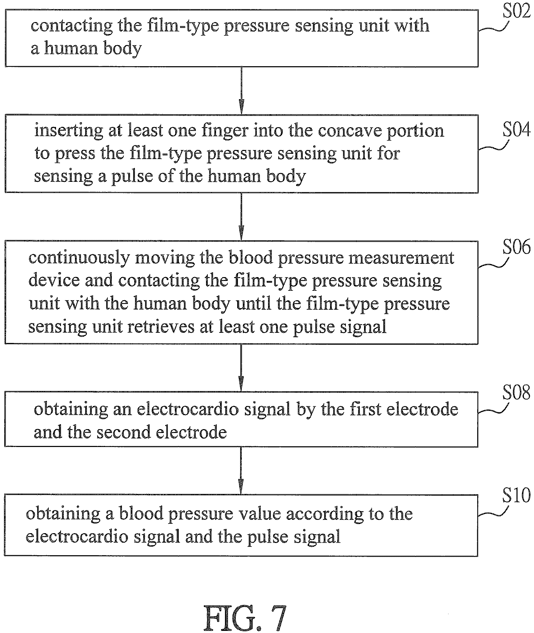

The present disclosure also discloses a blood pressure measurement method cooperated with any of the above mentioned blood pressure measurement devices. FIG. 7 is a flow chart of a blood pressure measurement method according to an embodiment of the disclosure. As shown in FIG. 7, the blood pressure measurement method includes the following steps of: contacting the film-type pressure sensing unit with a human body (step S02); inserting at least one finger into the concave portion to press the film-type pressure sensing unit for sensing a pulse of the human body (step S04); continuously moving the blood pressure measurement device and contacting the film-type pressure sensing unit with the human body until the film-type pressure sensing unit retrieves at least one pulse signal (step S06); obtaining an electrocardio signal by the first electrode and the second electrode (step S08); and obtaining a blood pressure value according to the electrocardio signal and the pulse signal (step S10).

FIG. 8 is a schematic diagram showing that the user moves the blood pressure measurement device to find the pulse. Referring to FIGS. 7 and 8, in the step S02, the user holds the blood pressure measurement device 10 and puts the film-type pressure sensing unit 110 to contact with a human body. In this embodiment, the user holds the blood pressure measurement device 10 with his/her right hand, and places the blood pressure measurement device 10 over the vessel C of the left wrist. Of course, the user may hold the blood pressure measurement device 10 with his/her left hand, and places the blood pressure measurement device 10 over the vessel C of the right wrist. As mentioned above, the film-type pressure sensing unit 110 may contact the another part of the human body, such as the wrist, arm, leg, neck, or the part near the rib of the trunk, and the disclosure is not restricted thereto.

In the step S04, the user inserts at least one finger A into the concave portion 101, holds the blood pressure measurement device 10, and places the blood pressure measurement device 10 on a part of the human body to press the film-type pressure sensing unit 110 for sensing a pulse (vibration) of the part of the human body. In this design, the finger A is usually the index finger. Of course, the finger A can be the middle finger or multiple fingers can be inserted into the concave portion 101 together based on the habit of the user. Since the finger is very sensitive, it can properly sense the pulse (vibration) through the film-type pressure sensing unit 110. In practice, the user may insert any part into the concave portion 101 that can sense the vibration (pulse) of the human body, and this disclosure is not limited. When the user inserts the finger A into the concave portion 101 to press the film-type pressure sensing unit 110, the film-type pressure sensing unit 110 and the first electrode 120 are pressed downwardly by a force F (see FIG. 3B), or the finger A may be affected by gravity to press the film-type pressure sensing unit 110 and the first electrode 120 to attach the human body.

In the step S06, the user may hold the blood pressure measurement device 10 and continuously moves the blood pressure measurement device 10, and the blood pressure measurement device 10 is contacted with the human body until the film-type pressure sensing unit 110 retrieves at least one pulse signal. In this embodiment, the user puts the blood pressure measurement device 10 over the vessel C of the wrist. If the finger does not sense any pulse, the user may move the blood pressure measurement device 10 or the wrist to make the film-type pressure sensing unit 110 to contact another position. If the finger does sense a pulse, the film-type pressure sensing unit 110 can make the strain gauge to generate deformation so as to cause the resistance change, which is transmitted to the processing unit 140. The processing unit 140 can calculate the pressure value according to the resistance change, and generate a pulse signal according to the continuous pressure changes.

In the step S08, the first electrode 120 and the second electrode 130 are provided to contact different parts of the human body. In this embodiment, the first electrode 120 is disposed under the pulse sensing surface 102 and contacted with the left wrist, and the second electrode 130 is annularly disposed on the left side surface of the blood pressure measurement device 10 and contacted with the right thumb. Accordingly, the first electrode 120 and the second electrode 130 can form a loop, and the processing unit 140 can cooperate with the potential difference measuring unit 150 to calculate and obtain the electrocardio signal.

During the measurement operation, the steps S06 and S08 for obtaining the pulse signal and the electrocardio signal, respectively, can be reversed, and this disclosure is not limited. In one embodiment, when the device is powered on, the first electrode 120 and the second electrode 130 are continuously in the detecting status. The user puts the blood pressure measurement device 10 to contact with the human body (e.g. finger or wrist), and the blood pressure measurement device 10 can determine whether the electrocardio signal is available or not. If the electrocardio signal is detected, the procedure for sensing the pulse signal is enabled. In another embodiment, when the device is powered on, the film-type pressure sensing unit 110 is continuously in the detecting status. The user presses the film-type pressure sensing unit 110 to contact against the human body for sensing the pulse signal of a vessel of any part of the human body. If the blood pressure measurement device 10 determines that the pulse signal is available, the procedure for sensing the electrocardio signal is enabled. In another embodiment, the body 100 of the blood pressure measurement device 10 may further include a power switch 191 (see FIG. 6). After the user turns on the power switch 191, the blood pressure measurement device 10 starts to detect and sense the pulse signal and the electrocardio signal.

In one embodiment, the body is configured with a calibration electrode. Accordingly, when the user holds the blood pressure measurement device, the second electrode and the calibration electrode are contacted with the hand of the user (step S09). In the step S09, the operated blood pressure measurement device can be that shown in FIGS. 5A to 5C, and the calibration electrode 180 is disposed on any position of the body 100 that allows the user to intuitively and easily touch. For example, the calibration electrode 180 can be disposed on the annular side surface or the inner wall of the concave portion 101. The configuration of the calibration electrode 180 can filter the undesired noise signal away. In addition, the calibration electrode 180 and the first electrode 120 may generate a calibration value for averaging with the electrocardio signal generated by the first electrode 120 and the second electrode 130, thereby obtaining a more precise electrocardio signal. In one embodiment, the calibration electrode 180 is annularly disposed on the right side surface of the blood pressure measurement device 10 opposite to the second electrode 130. When the user holds the blood pressure measurement device 10, the thumb and middle finger may contact with the second electrode 130 and the calibration electrode 180, respectively. The potential difference measuring unit 150 (see FIG. 2) measures the first electrode 120 and the calibration electrode 180 for at least twice during a period, and the at least two measuring results are transmitted to the processing unit 140. Then, the processing unit 140 calculates a first calibration value and a second calibration value according to the received measuring results. Afterwards, the processing unit 140 averages the first calibration value and the second calibration value so as to calibrate the electrocardio signal generated by the first electrode 120 and the second electrode 130. The above-mentioned calibration procedure takes the average of the calibration values as a reference for modifying or revoking the improper values, such as the maximum value and the minimum value. In addition, it is possible to filter the noise signal by capacitors for performing the desired calibration to obtain a more reliable electrocardio signal.

Finally, the step S10 is to obtain a blood pressure value according to the electrocardio signal and the pulse signal. Referring to FIGS. 2 and 6, the processing unit 140 can record the time for obtaining the electrocardio signal (calculated by the potential difference measuring unit 150) and the time for obtaining the pulse signal (calculated by the film-type pressure sensing unit 110), and then calculate the pulse transmission time (PTT) according to the difference of the recorded times. In detail, the PTT is the difference between the time of appearance of the R wave (corresponding to the medium term of depolarization of the ventricular) of the electrocardio signal being judged in one heartbeat, and the time of occurrence of the pulsation of the radial artery measured on the to-be-measured part of the body (the left wrist in this embodiment). In other words, the PTT is the time duration when the pulse generated in one heartbeat travels from the heart to the left wrist. The distance from the heart to the left wrist may be a predetermined value, or may be obtained by multiplying the height of the user (manually inputted) with a parameter. Then, the distance is divided by the PTT to obtain the pulse wave velocity (PWV), which is the velocity of the pulse that is generated by the systole and reaches the left wrist. Thereafter, the processing unit 140 can calculate the user's blood pressures, comprising the systolic pressure and the diastolic pressure, according to the PWV through the algorithm. In this embodiment, the processing unit 140 may transmit the measured result to the display unit 160 for display. Accordingly, after the user finishes the blood pressure measurement, he/she can simply turn over the blood pressure measurement device 10 to immediately view the measuring results such as the blood pressure value. This design is intuitive and very convenient. Of course, in other embodiments, the blood pressure measurement device 10 has a communication unit, which transmits the measured result to a smart phone or a tablet computer for display through wireless communication, such as Bluetooth, 3G/4G mobile communication protocol or Wi-Fi. However, the invention is not restricted thereto.

In summary, the blood pressure measurement device and method of the disclosure can measure the pulse signal and the electrocardio signal for calculating the blood pressure value without utilizing the cuff for pressurization and depressurization. Accordingly, the size of the device can be minimized to carry out the easy carrying issue and enhance the convenience in measuring the blood pressure so as to achieve the purpose of monitoring the blood pressure at any time. More important, the concave portion is communicated with the pulse sensing surface, so that the user can easily and precisely find the pulse position (assisted by finger) for measurement so as to place the film-type pressure sensing unit on the measuring position. In some embodiments, the present disclosure can easily place the device on the measuring position when measuring the signals. This is an intuitional operation for precisely measuring the blood pressure, and the operation procedure is simple so that the users of any ages can easily operate it.

Although the invention has been described with reference to specific embodiments, this description is not meant to be construed in a limiting sense. Various modifications of the disclosed embodiments, as well as alternative embodiments, will be apparent to persons skilled in the art. It is, therefore, contemplated that the appended claims will cover all modifications that fall within the true scope of the invention.

## Claims

1. A blood pressure measurement device, comprising:
a body comprising a pulse sensing surface and a concave portion;
a film-type pressure sensing unit disposed on the pulse sensing surface and communicated with the concave portion;
a first electrode disposed on the pulse sensing surface; and
a second electrode disposed on the body corresponding to the first electrode, wherein the first electrode and second electrode are a pair of ECG electrodes.

2. The blood pressure measurement device of claim 1, further comprising:
a calibration electrode disposed on the body.

3. The blood pressure measurement device of claim 2, wherein the calibration electrode and the second electrode are disposed at two sides of the pulse sensing surface, respectively.

4. The blood pressure measurement device of claim 1, wherein the second electrode is disposed on an inner wall of the concave portion.

5. The blood pressure measurement device of claim 1, wherein the second electrode is disposed adjacent to at least one side of the pulse sensing surface.

6. The blood pressure measurement device of claim 1, wherein an inner wall of the concave portion comprises a slant surface.

7. The blood pressure measurement device of claim 1, wherein a waterproof material is configured on an inner wall of the concave portion.

8. The blood pressure measurement device of claim 1, wherein the first electrode and the film-type pressure sensing unit are overlapped, and an area of the first electrode is greater than an area of the film-type pressure sensing unit.

9. The blood pressure measurement device of claim 1, wherein the first electrode is disposed around the film-type pressure sensing unit.

10. A blood pressure measurement method cooperated with a blood pressure measurement device, wherein the blood pressure measurement device comprises a body, a film-type pressure sensing unit, a first electrode and a second electrode, the body comprises a pulse sensing surface and a concave portion, the film-type pressure sensing unit is disposed on the pulse sensing surface and communicated with the concave portion, the first electrode is disposed on the pulse sensing surface, the second electrode is disposed on the body corresponding to the first electrode, and the first electrode and second electrode are a pair of ECG electrodes, the blood pressure measurement method comprising steps of:
contacting the film-type pressure sensing unit with a human body;
inserting at least one finger into the concave portion to press the film-type pressure sensing unit for sensing a pulse of the human body;
continuously moving the blood pressure measurement device and contacting the film-type pressure sensing unit with the human body until the film-type pressure sensing unit retrieves at least one pulse signal;
obtaining an electrocardio signal by the first electrode and the second electrode; and
obtaining a blood pressure value according to the electrocardio signal and the pulse signal.

11. The blood pressure measurement method of claim 10, wherein when the film-type pressure sensing unit contacts with the human body, the first electrode also contacts with the human body.

12. The blood pressure measurement method of claim 10, wherein when a user holds the blood pressure measurement device by a hand, the second electrode is in contact with the hand of the user.

13. The blood pressure measurement method of claim 10, wherein the blood pressure measurement further comprises a calibration electrode disposed on the body, and the second electrode and the calibration electrode are in contact with a hand of the user when the user holds the blood pressure measurement device by the hand.

14. The blood pressure measurement method of claim 10, wherein when the first electrode and the second electrode obtain the electrocardio signal, the blood pressure measurement device is immediately enabled to measure the pulse signal.

15. The blood pressure measurement method of claim 10, wherein after the film-type pressure sensing unit senses the pulse signal, the blood pressure measurement device is immediately enabled to measure the electrocardio signal.
